**Europäisches Patentamt**

**European Patent Office**

**Office européen des brevets**

(19)

(11) Veröffentlichungsnummer: **0 107 783**
**B1**

(12) ## EUROPÄISCHE PATENTSCHRIFT

(45) Veröffentlichungstag der Patentschrift:
08.04.87

(51) Int. Cl.⁴: **B 01 D 53/14, C 10 K 1/14**

(21) Anmeldenummer: **83109425.5**

(22) Anmeldetag: **22.09.83**

(54) **Verfahren zum Entfernen von CO2 und/oder H2S aus Gasen.**

(30) Priorität: **02.10.82 DE 3236601**

(43) Veröffentlichungstag der Anmeldung:
**09.05.84 Patentblatt 84/19**

(45) Bekanntmachung des Hinweises auf die Patenterteilung:
**08.04.87 Patentblatt 87/15**

(84) Benannte Vertragsstaaten:
**AT BE DE FR GB IT NL SE**

(56) Entgegenhaltungen:
**DE-A-2 551 717**
**DE-C-1 494 781**

**CHEMIE-INGENIEUR-TECHNIK, Band 53, Nr. 2,
1981, Verlag Chemie, Weinheim, D.WERNER
"Gasreinigungsverfahren für grosse Wasserstoff-
Mengen", Seiten 73-81**

(73) Patentinhaber: **BASF Aktiengesellschaft, Carl-
Bosch- Strasse 38, D-6700 Ludwigshafen (DE)**

(72) Erfinder: **Volkamer, Klaus, Dr., Heidelberger Ring
21, D-6710 Frankenthal (DE)**
Erfinder: **Wagner, Eckhart, Dr., Jakobsgarten,
D-6700 Ludwigshafen (DE)**
Erfinder: **Wagner, Ulrich, Dr., Knospstrasse 7,
D-6703 Limburgerhof (DE)**

EP 0 107 783 B1

## Beschreibung

Die vorliegende Erfindung betrifft ein Verfahren zum Entfernen von $CO_2$ und/oder $H_2S$ aus Gasen mittels einer wäßrigen Absorptionsflüssigkeit.

Es ist bekannt, z.B. aus A.L. Kohl - F.C. Riesenfeld, Gas Purification, 3. Auflage, 1979, wäßrige Lösungen von Monoethanolamin oder Diethanolamin oder Mischungen von Cyclotetramethylensulfon und einer wäßrigen Lösung von Diisopropanolamin als Lösungsmittel zur Entfernung von $CO_2$ und/oder $H_2S$ aus Gasen zu verwenden. Bei diesen Verfahren ist es erforderlich, das mit $CO_2$ und gegebenenfalls $H_2S$ beladene Lösungsmittel in einer Ausstreifkolonne durch Zufuhr von Dampf zu regenerieren, was mit erheblichem Energieaufwand verbunden ist. Bei der Entfernung von $CO_2$ und gegebenenfalls $H_2S$ aus höhere Kohlenwasserstoffe enthaltenden Erdgasen mittels einer Mischung von Cyclotetramethylensulfon und einer wäßrigen Lösung von Diisopropanolamin kommt als zusätzlicher Nachteil hinzu, daß die höheren Kohlenwasserstoffe in diesem Lösungsmittel eine relativ hohe Löslichkeit aufweisen, so daß das am Kopf der Ausstreifkolonne abgezogene Sauergas einen relativ hohen Gehalt an Kohlenwasserstoffen aufweist, der, falls das Sauergas $H_2S$ enthält, zu Schwierigkeiten in einer nachgeschalteten Claus-Anlage führen kann. Primäre oder sekundäre Alkanolamine, wie Monoethanolamin oder Diethanolamin, können außerdem nur als wäßrige Lösungen mit verhältnismäßig geringer Konzentration an diesen Alkanolaminen verwendet werden, da bei Verwendung höherer Konzentrationen schwere Korrosionsschäden an Anlageteilen auftreten können.

Weiter ist aus der DE-A-25 51 717 ein Verfahren zur Entfernung von $CO_2$ und/oder $H_2S$ aus Gasen unter Verwendung von Piperazin enthaltenden chemischen oder physikalischen Lösungsmitteln bekannt. Bei den chemischen Lösungsmitteln wird auch die Verwendung von Methyldiethanolamin (MDEA) beschrieben. Bei dem Verfahren wird das in einer einstufigen Wäsche aus der Absorptionsstufe erhaltene beladene Lösungsmittel zunächst durch Entspannung regeneriert und dann weiter regeneriert in einer zweiten Stufe in einer Ausstreifzone. Dieses Verfahren weist einen relativ hohen Dampfverbrauch auf.

Außerdem wird in Chemie-Ingenieur-Technik Band 53, Nr. 2, 1981, Seiten 73 - 81 ein Verfahren zum Entfernen von $CO_2$ aus Gasen beschrieben, bei dem in einer zweistufigen chemischen Wäsche das beladene Lösungsmittel zunächst in zwei Entspannungsstufen entspannt und anschließend in einem Ausstreifer weiter regeneriert wird.

Weiter ist aus DE-C-1 494 781 eine zweistufige Gaswäsche bekannt, bei der das Verfahren mit zwei verschiedenen Lösungsmitteln betrieben werden muß, indem in einer ersten Absorptionsstufe eine wäßrige Lösung eines Trialkanolamins und in einer zweiten Absorptionsstufe eine wäßrige Lösung von aminosauren Alkalisalzen verwendet wird.

Schließlich wird in EP-A-107 046 (Priorität: 02.10.1982; Anmeldetag: 22.09.1983; Veröffentlichungstag: 02.05.1984) ein Verfahren zum Entfernen von $CO_2$ und gegebenenfalls $H_2S$ aus Gasen mittels einer Methyldiethanolamin enthaltenden wäßrigen Absorptionsflüssigkeit in einer einstufigen Wäsche beschrieben, bei dem die Regenerierung der beladenen Absorptionsflüssigkeit ausschließlich in einer oder mehreren Entspannungsstufen durchgeführt wird.

Es bestand daher Bedarf nach einem Verfahren zum Entfernen von $CO_2$ und/ oder $H_2S$ aus Gasen, bei dem die Nachteile der bekannten Verfahren vermieden werden können.

Es wurde nun ein vorteilhaftes Verfahren gefunden zum Entfernen von $CO_2$ und/oder $H_2S$ aus $CO_2$ enthaltenden Gasen, bei dem man die $CO_2$ und/oder $H_2S$ enthaltenden Gase in einer Absorptionsstufe bei Temperaturen von 40 bis 100° C mit einer 20 bis 70 Gew.-% Methyldiethanolamin enthaltenden wäßrigen Absorptionsflüssigkeit behandelt, am Kopf der Absorptionsstufe die behandelten Gase abzieht, am Sumpf der Absorptionsstufe die mit $CO_2$ und/oder $H_2S$ beladene wäßrige Absorptionsflüssigkeit abzieht und anschließend regeneriert und die regenerierte Absorptionsflüssigkeit in die Absorptionsstufe zurückführt, welches dadurch gekennzeichnet ist, daß man die Regenerierung ausschließlich in einer oder mehreren Entspannungsstufen durchführt und dabei am Kopf der Entspannungsstufe bzw. Entspannungsstufen die Entspannungsgase abzieht, zum Ausgleich der Wasserverluste durch in den am Kopf der Absorptionsstufe und der Entspannungsstufe bzw. Entspannungsstufen abgezogenen Gasströmen enthaltenes Wasser am Sumpf der letzten Entspannungsstufe bzw., falls nur eine Entspannungsstufe verwendet wird, am Sumpf dieser einen Entspannungsstufe eine dem Wasserverlust entsprechende Menge Wasserdampf zuführt.

Nach dem neuen Verfahren wird das mit $CO_2$ und/oder $H_2S$ beladene Lösungsmittel ohne Verwendung einer Ausstreifkolonne allein durch Entspannung in einer oder mehreren Entspannungsstufen regeneriert, so daß eine erhebliche Reduktion sowohl der Investitionskosten als auch der Energieverbrauchskosten erreicht wird. Weiter können bei dem neuen Verfahren relativ hohe Methyldiethanolaminkonzentrationen in der Absorptionsflüssigkeit verwendet werden, ohne daß es zu Korrosionsschäden in der Gaswaschanlage kommt. Ein weiterer Vorteil des Verfahrens besteht darin, daß Wasserverluste, die in Gaswaschanlagen durch in den am Kopf der Absorptionskolonne und der Entspannungsbehälter abgezogenen Gasströmen enthaltenes Wasser entstehen, durch Zuführung einer dem Wasserverlust entsprechenden Menge Wasserdampf am Sumpf der letzten Entspannungsstufe ausgeglichen werden. Durch diese Arbeitsweise kann neben der Regelung des Wasserhaushaltes der Gaswaschanlage gleichzeitig der Wärmehaushalt der Gaswaschanlage geregelt werden, so daß ein für die Regelung des Wärmehaushaltes in der Gaswaschanlage vorhandener Wärmetauscher kleiner ausgeführt werden oder ggf. ganz wegfallen kann.

Als nach dem erfindungsgemäßen Verfahren zu behandelnde Gase kommen beispielsweise Kohlevergasungsgase, Synthesegase, Koksofengase und vorzugsweise Erdgase in Betracht. Vorteilhaft wird das Verfahren zum Entfernen von $CO_2$ und/oder $H_2S$ aus Erdgasen verwendet, die neben Methan noch höhere Kohlrnwasserstoffe enthalten. Bei diesen höheren Kohlenwasserstoffen handelt es sich im allgemeinen um $C_2$- bis $C_{30}$-Kohlenwasserstoffe, vorzugsweise $C_2$- bis $C_{20}$-Kohlenwasserstoffe, insbesondere $C_2$- bis $C_{12}$-Kohlenwasserstoffe, die in der Regel aliphatisch sind, z.B. Ethan, Propan, Isobutan, n-Butan, Isopentan, n-Pentan, die Hexane, Heptane, Octane, Nonane, Decane und die höheren Homologen. Die höheren Kohlenwasserstoffe können neben den aliphatischen Kohlenwasserstoffen auch noch aromatische Kohlenwasserstoffe wie Benzol enthalten. Im allgemeinen beträgt der Gehalt der Erdgase an den höheren Kohlenwasserstoffen 0.1 bis 40 Mol.-%, vorzugsweise 0.5 bis 30 Mol.-%, insbesondere 1 bis 20 Mol.-%.

Die Gase weisen im allgemeinen einen $CO_2$-Gehalt von 1 bis 90 Mol.-%, vorzugsweise 2 bis 90 Mol.-%, insbesondere 5 bis 60 Mol.-% auf. Neben dem $CO_2$ können die Gase sls weiteres Sauergas $H_2S$ enthalten oder sie können $H_2S$ allein enthalten, z.B. in Mengen vom wenigen Mol.-ppm, beispielsweise 1 Mol.-ppm, bis 50 Mol.%.

Als Lösungsmittel wird für das erfindungsgemäße Verfahren eine 20 bis 70 Gew.%, vorzugsweise 30 bis 65 Gew.%, insbesondere 40 bis 60 Gew.% Methyldiethanolamin enthaltende wäßrige Absorptionsflüssigkeit verwendet. Zweckmäßig wird eine wäßrige Methyldiethanolaminlösung eingesetzt, z.B. eine wäßrige Lösung von Methyldiethanolamin technischer Reinheit. In einer vorteilhaften Ausführungsform des Verfahrens verwendet man eine wäßrige Methyldiethanolaminlösung, die zusätzlich 0,1 bis 1 Mol/l, vorzugsweise 0,2 bis 0,8 Mol/l, insbesondere 0,25 bis 0,6 Mol/l eines sekundären Amins oder Alkanolamins, vorzugsweise Methylmonoethanolamin, ganz besonders vorteilhaft Piperazin, enthält. Das erfindungsgemäße Verfahren wird in der Weise durchgeführt, daß das $CO_2$ und/oder $H_2S$ enthaltende Gas zunächst in einer Absorptionsstufe mit der Methyldiethanolamin enthaltenden Absorptionsflüssigkeit behandelt wird. Dabei werden in der Absorptionsstufe Temperaturen von 40 bis 100°C, vorzugsweise 50 bis 90°C, insbesondere 60 bis 80°C für die Absorptionsflüssigkeit aufrechterhalten. Im allgemeinen werden in der Absorptionsstufe Drucke von 10 bis 110 bar, vorzugsweise 20 bis 100 bar, insbesondere 30 bis 90 bar angewendet. Als Absorptionsstufe wird zweckmäßig eine Absorptionskolonne verwendet, im allgemeinen eine Füllkörperkolonne oder eine mit Böden ausgestattete Kolonne. Zweckmäßig wird das zu behandelnde Gas am Sumpf und die Absorptionsflüssigkeit am Kopf der Absorptionskolonne zugeführt, wobei die sauren Gase $CO_2$ und/oder $H_2S$ im Gegenstrom ausgewaschen werden. Während gegebenenfalls vorhandenes $H_2S$ zweckmäßig weitgehend, im allgemeinen auf $H_2S$-Gehalte im behandelten Gas von höchstens 120 Mol.-ppm, vorzugsweise höchstens 10 Mol.-ppm, insbesondere höchstens 3 Mol.-ppm ausgewaschen wird, kann es vorteilhaft sein, das $CO_2$ im Gas soweit auszuwaschen, daß die $CO_2$-Gehalte im behandelten Gas höchstens etwa 0,5 bis 6, vorzugsweise 0,5 bis 5, insbesondere 1 bis 4 Mol.% betragen. Das behandelte Gas wird zweckmäßig am Kopf der Absorptionsstufe, zweckmäßig an einen Punkt, der oberhalb der Zuführung der Absorptionsflüssigkeit liegt, abgezogen. Die mit den Sauergasen $CO_2$ und/oder $H_2S$ beladene Absorptionsflüssigkeit wird zweckmäßig am Sumpf der Absorptionszone abgezogen.

Anschließend wird die beladene Absorptionsflüssigkeit in ein oder mehreren Entspannungsstufen regeneriert. Dabei wird in der letzten Entspannungsstufe bzw., falls nur eine Entspannungsstufe verwendet wird, in dieser einzelnen Entspannungsstufe zweckmäßig auf einen Druck von etwa 1 bis 3 bar, vorzugsweise 1 bis 1,8 bar, insbesondere 1 bis 1,5 bar entspannt. Es kann auch zweckmäßig sein, die letzte Entspannungsstufe bzw. die einzelne Entspannungsstufe unter vermindertem Druck gegenüber Atmosphärendruck, z.B. bei Drucken von 0,5 bis etwa 1 bar, vorzugsweise von 0,8 bis etwa 1 bar, zu betreiben. Es kann vorteilhaft sein, mindestens zwei Entspannungsstufen, z.B. 2 bis 5, vorzugsweise 2 oder 3, insbesondere 2 Entspannungsstufen, für die Regenerierung der beladenen Absorptionsflüssigkeit zu verwenden. Vorzugsweise wird dabei das beladene Absorptionsmittel in der ersten Entspannungsstufe nach der Absorptionsstufe auf einen Druck entspannt, der mindestens 5 bar beträgt. Es kann dabei vorteilhaft sein, daß der Druck, auf den in der ersten Entspannungsstufe nach der Absorptionsstufe entspannt wird, zusätzlich mindestens der Summe der Partialdrucke an $CO_2$ und/oder $H_2S$ in dem der Absorptionsstufe zugeführten, $CO_2$ und/oder $H_2S$ enthaltenden Gas ist. Bei dieser Arbeitsweise können die Verdampfung von Wasser und die damit verbundenen Energieverluste sowie die Verluste an Kohlenwasserstoffen besonders niedrig gehalten werden. Für die Entspannung werden zweckmäßig Entspannungsbehälter, die z.B. auch als Kolonnen gestaltet sein können, verwendet. Diese Entspannungsbehälter können frei von besonderen Einbauten sein. Es können jedoch auch mit Einbauten, z.B. mit Füllkörpern ausgestattete Kolonnen verwendet werden.

In der Regel wird zum Ausgleich von verfahrensbedingten Wärmeverlusten, die beispielsweise durch die Entspannungsverdampfung bewirkt werden, dem Verfahren Wärme zugeführt. Dies erfolgt zweckmäßig in einer vor die letzte Entspannungsstufe bzw., falls nur eine Entspannungsstufe verwendet wird, vor diese einzelne Entspannungsstufe geschalteten Wärmeaustauschzone, in der die beladene Absorptionsflüssigkeit vor der Entspannung in der letzten bzw. einzelnen Entspannungsstufe erwärmt wird. In der Regel wird die Absorptionsflüssigkeit in der Austauschzone um maximal 20°C erwärmt, wobei die Absorptionsflüssigkeit im allgemeinen auf eine Temperatur von höchstens 90°C, in der Regel von höchstens 85°C erwärmt wird. Für die Wärmeaustauschzone werden im allgemeinen Wärmetauscher, z.B. ein Röhrenwärmetauscher, verwendet.

Zum Ausgleich der Wasserverluste, die bei dem Verfahren durch in den am Kopf der Absorptionsstufe und der Entspannungsstufe bzw. Entspannungsstufen abgezogenen Gasströmen enthaltenes Wasser in den Gasströmen entstehen, wird am Sumpf der letzten Entspannungsstufe bzw., falls nur eine Entspannungsstufe verwendet wird, am Sumpf dieser einen Entspannungsstufe eine dem Wasserverlust entsprechende Menge Wasserdampf zugeführt. In der Regel wird das in den abgezogenen Gasströmen enthaltene Wasser im wesentlichen als Wasserdampf abgezogen. Dem Sumpf der Entspannungsstufe kann Niederdruck-, Mitteldruck- oder Hochdruckdampf, d.h. z.B. 1- bis 100-bar-Dampf zugeführt werden. Vorzugsweise verwendet man Dampf im niederen Druckbereich, z.B. 1,5- bis 10-bar-Dampf, vorteilhaft 1,5- bis 5-bar-Dampf, da dieser Niederdruckdampf im allgemeinen wohlfeil zur Verfügung steht. Durch die erfindungsgemäße Dampfzufuhr zur Entspannungsstufe kann neben der Regelung des Wasserhaushaltes gleichzeitig der Wärmehaushalt der Gaswaschanlage geregelt werden. In einer bevorzugten Ausführungsform des Verfahrens wird dementsprechend zum Ausgleich von verfahrensbedingten Wärmeverlusten dem Verfahren entweder gleichzeitig Wärme in der vor die letzte Entspannungsstufe bzw., falls nur eine Entspannungsstufe verwendet wird, vor diese eine Entspannungsstufe geschalteten Wärmeaustauschzone sowie mittels des am Sumpf der letzten Entspannungsstufe bzw. der einzelnen Entspannungsstufe zugegebenen Wasserdampfs zugeführt oder Wärme allein mittels des am Sumpf der letzten Entspannungsstufe bzw. der einzelnen Entspannungsstufe zugegebenen Wasserdampfs zugeführt.

Die Sauergase $CO_2$ und/oder $H_2S$ werden zweckmäßig am Kopf der letzten Entspannungsstufe abgezogen. Falls das abgezogene Sauergas $H_2S$ enthält, wird es zweckmäßig durch Oxidation des $H_2S$, z.B. in einer Claus-Anlage, aufgearbeitet. Die am Sumpf der letzten Entspannungsstufe abgezogene regenerierte Absorptionsflüssigkeit wird in die Absorptionszone zurückgeführt.

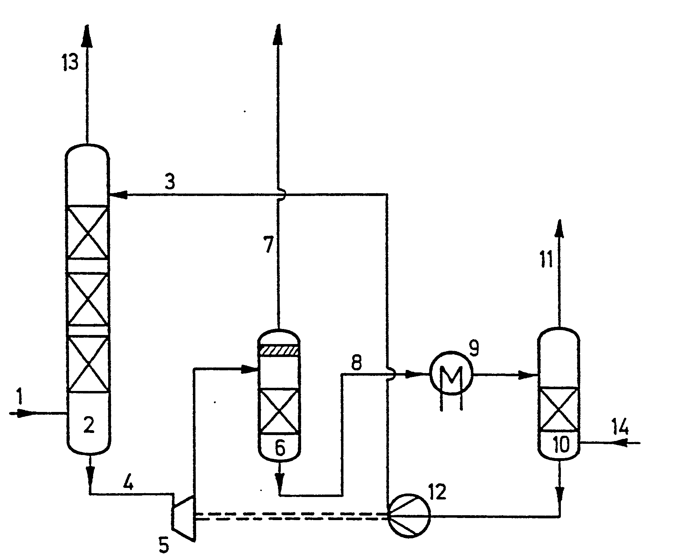

Nachfolgend werden weitere Einzelheiten der Erfindung anhand eines Ausführungsbeispiels, dessen Verfahrensablauf in der Figur schematisch dargestellt ist, erläutert.

Ein $CO_2$ und/oder $H_2S$ enthaltendes Gas, z.B. ein höhere Kohlenwasserstoffe, z.B. aliphatische $C_2$- bis $C_{10}$-Kohlenwasserstoffe enthaltendes Erdgas, wird über Leitung 1 unter Druck in den Sumpf der Absorptionskolonne 2 gegeben. Gleichzeitig wird über Leitung 3 als Absorptionsflüssigkeit 20 bis 70 gew.-%ige wäßrige Methyldiethanolaminlösung auf den Kopf der Absorptionskolonne gegeben. Die Absorptionsflüssigkeit, die im Gegenstrom zum Gas geführt wird, belädt sich mit den sauren Gasen $CO_2$ und/oder $H_2S$, und die beladene Absorptionsflüssigkeit wird über Leitung 4 am Sumpf der Absorptionskolonne abgezogen. Das gewaschene Gas wird über Leitung 13 am Kopf der Absorptionskolonne abgezogen. Der beladene Absorptionsflüssigkeitsstrom 4 wird anschließend in einen Entspannungsverdampfungsbehälter 6 zweckmäßig auf einen Druck von mindestens 5 bar entspannt, beispielsweise über ein Ventil oder vorzugsweise über eine Entspannungsturbine 5. Hierbei wird ein Kohlenwasserstoffe und $CO_2$ enthaltendes Zwischenentspannungsgas aus der Absorptionsflüssigkeit freigesetzt, das über Leitung 7 am Kopf des Entspannungsbehälters 6 abgezogen wird. Am Boden des Entspannungsbehälters 6 wird die teilentspannte Absorptionsflüssigkeit über Leitung 8 abgezogen, in Wärmetauscher 9 erwärmt, z.B. um 1 bis 15°C, und die erwärmte Absorptionsflüssigkeit wird in einen zweiten Entspannungsbehälter 10 entspannt, z.B. auf einen Druck von 1 bis 2 bar. Hierbei wird ein $CO_2$-reiches Entspannungsgas, z.B. mit einer $CO_2$-Konzentration von 98 Mol.-%, freigesetzt, das über Leitung 11 am Kopf des Entspannungsbehälters 10 abgezogen wird. In den Sumpf des Entspannungsbehälters 10 wird zum Ausgleich der Wasserverluste des Systems über Leitung 14 Wasserdampf, z.B. 2,5 bar-Niederdruckdampf, eingeleitet. Die am Sumpf des Entspannungsbehälters 10 abgezogene regenerierte Absorptionsflüssigkeit wird mit Hilfe einer Umlaufpumpe 12 zum Kopf der Absorptionskolonne 2 zurückgeführt.

Das folgende Beispiel veranschaulicht die Erfindung.

**Beispiel**

In einer Absorptionskolonne werden 3,15 kmol/h eines $CO_2$-haltigen Erdgases mit einer 50 gew.%igen wäßrigen Methyldiethanolamin-Lösung als Absorptionsflüssigkeit bei einem Druck von 75 bar gewaschen. Das zu reinigende Gas hat folgende Zusammensetzung:

| | |
|---|---|
| $CO_2$ | 10,0 Mol.% |
| $CH_4$ | 75,0 Mol.% |

höhere Kohlenwasserstoffe ($C_2$- bis $C_{12}$-Kohlen-wasserstoffe) 15,0 Mol.%

Die Temperatur des Absorptionsmittels im Zulauf zur Absorptionskolonne beträgt 70°C. Der $CO_2$-Gehalt im gewaschenem Gas beträgt 2,0 Mol.%. Das die Absorptionskolonne verlassende beladene Waschmittel wird in einem ersten Entspannungsbehälter auf einen Druck von 20 bar entspannt. Hierbei werden 0,04 kmol/l eines kohlenwasserstoffreichen Zwischenentspannungsgases mit einer $CO_2$-Konzentration von 34,3 Mol.% aus der Lösung freigesetzt und am Kopf des ersten Entspannungsbehälters abgezogen. Die teilentspannte Absorptionsflüssigkeit wird anschließend in einem Wärmetauscher erwärmt. Die erwärmte Absorptionsflüssigkeit wird in einem zweiten Entspannungsbehälter auf 1,3 bar entspannt. Hierbei werden 0,241 kmol/h eines $CO_2$-reichen Entspannungsgases mit einer $CO_2$-Konzentration von 97,55 Mol.%, einer Methankonzentration von 1,68 Mol.% und einer Konzentration an höheren Kohlenwasserstoffen vom 0,77 Mol.% frei, die am Kopf des zweiten Entspannungsbehälters abgezogen werden. In den Sumpf des zweiten Entspannungsbehälters wird zum Ausgleich der Wasserverluste des Systems Niederdruckdampf (2,5-bar-Dampf) eingeleitet. Die am Sumpf des Entspannungsbehälters abgezogene Absorptionsflüssigkeit wird mit Hilfe einer Umlaufpumpe zum Kopf der Absorptionskolonne zurückgepumpt.

**Patentansprüche**

1. Verfahren zum Entfernen von $CO_2$ und/oder $H_2S$ aus $CO_2$ und/oder $H_2S$ enthaltenden Gasen, bei dem man die $CO_2$ und/oder $H_2S$ enthaltenden Gase in einer Absorptionsstufe bei Temperaturen von 40 bis 100°C mit einer 20 bis 70 Gew.-% Methyldiethanolamin enthaltenden wäßrigen Absorptionsflüssigkeit behandelt, am Kopf der Absorptionsstufe die behandelten Gase abzieht, am Sumpf der Absorptionsstufe die mit $CO_2$ und/oder $H_2S$ beladene wäßrige Absorptionsflüssigkeit abzieht und anschließend regeneriert und die regenerierte Absorptionsflüssigkeit in die Absorptionsstufe zurückführt, dadurch gekennzeichnet, daß man die Regenerierung ausschließlich in einer oder mehreren Entspannungsstufen durchführt und dabei am Kopf der Entspannungsstufe bzw.

Entspannungsstufen die Entspannungsgase abzieht und zum Ausgleich der Wasserverluste durch in den am Kopf der Absorptionsstufe und der Entspannungsstufe bzw. Entspannungsstufen abgezogenen Gasströmen enthaltenes Wasser am Sumpf der letzten Entspannungsstufe bzw., falls nur eine Entspannungsstufe verwendet wird, am Sumpf dieser einen Entspannungsstufe eine dem Wasserverlust entsprechende Menge Wasserdampf zuführt.

2. Verfahren nach Anspruch 1, dadurch gekennzeichnet, daß zum Ausgleich von verfahrensbedingten Wärmeverlusten gleichzeitig Wärme in einer vor die letzte Entspannungsstufe bzw., falls nur eine Entspannungsstufe verwendet wird, vor diese eine Entspannungsstufe geschalteten Wärmeaustauschzone sowie mittels des am Sumpf der letzten Entspannungsstufe bzw. der einzelnen Entspannungsstufe zugegebenen Wasserdampfs zugeführt wird.

**Claims**

1. A process for removing $CO_2$ and/or $H_2S$ from a gas which contains $CO_2$ and/or $H_2S$, in which the gas containing $CO_2$ and/or $H_2S$ is treated, in an absorption stage at from 40 to 100°C, with an aqueous absorption liquid containing from 20 to 70% by weight of methyldiethanolamine, the treated gas is taken off at the top of the absorption stage, the aqueous absorption liquid laden with $CO_2$ and/ or $H_2S$ is taken off at the bottom of the absorption stage and then regenerated, and the regenerated absorption liquid is recycled to the absorption stage, characterized in that the regeneration is carried out exclusively in one or more flash stages, the flash gases being taken off at the top of the flash stage or flash stages, and, to compensate for the losses of water as a result of water present in the gas streams taken off at the top of the absorption stage and of the flash stage or flash stages, an amount of steam corresponding to the water loss is fed in at the bottom of the last flash stage or, if only one flash stage is used, at the bottom of this stage.

2. A process as claimed in claim 1, characterized in that, to compensate for heat losses due to the process, heat is supplied to a heat exchange zone located upstream of the last flash stage or, if only one flash stage is used, upstream of this stage and, at the same time, by means of the steam fed in at the bottom of the last flash stage or of the single stage.

**Revendications**

1. Procédé d'extraction du $CO_2$ et(ou) du $H_2S$ de mélanges gazeux en contenant, dans lequel on traite les mélanges gazeux contenant du $CO_2$

et(ou) du $H_2S$ dans un stade d'absorption, à des températures comprises entre 40 et 100°C, avec un liquide d'absorption aqueux, contenant entre 20 et 70 % en poids de méthyl-diéthanolamine et soutire en tête du stade d'absorption les produits gazeux traités et comme résidu du stade d'absorption un liquide d'absorption aqueux chargé de $CO_2$ et(ou) de $H_2S$, ce liquide d'absorption étant régénéré, puis recyclé dans le stade d'absorption, caractérisé en ce que la régénération est réalisée exclusivement dans un ou plusieurs stades de détente, les produits gazeux libérés par la détente dans le ou les stades de détente étant soutirés en tête de celui-ci ou de chacun de ceux-ci, tandis que les pertes en eau, dues à l'entraînement d'eau par les courants de produits gazeux soutirés en tête du stade d'absorption et du ou des stades de détente, sont compensées par l'introduction dans le fond du stade de détente ou du dernier stade de détente, lorsqu'il y en a plusieurs, d'une quantité de vapeur d'eau correspondant à la perte d'eau.

2. Procédé suivant la revendication 1, caractérisé en ce que les pertes de chaleur dues au procédé sont compensées par l'introduction simultanée de chaleur dans une zone d'échange de chaleur disposée en amont du stade de détente ou du dernier stade de détente, s'il y en a plusieurs, ainsi que par la vapeur d'eau introduite dans le fond du stade de détente ou du dernier stade de détente, lorsqu'il y en a plusieurs.